# FASCICULE DE BREVET EUROPEEN

(11) **EP 1 560 524 B1**
(45) Date de publication et mention de la délivrance du brevet: **27.05.2009**
(21) Numéro de dépôt: 03780292.3
(22) Date de dépôt: 15.10.2003
(51) Int. Cl.: A61B 17/00, A61B 17/12

(54) **DISPOSITIF OCCLUSIF A DESTINATION MEDICALE OU CHIRURGICALE**
VERSCHLUSSVORRICHTUNG FÜR CHIRURGISCHE ODER MEDIZINISCHE ZWECKE
OCCLUSIVE DEVICE FOR MEDICAL OR SURGICAL USE

(30) Priorité: 15.11.2002 FR 0214290
(43) Date de publication de la demande: 10.08.2005
(73) Titulaire: Mialhe, Claude, 83300 Draguignan (FR)
(72) Inventeur: Mialhe, Claude, 83300 Draguignan (FR)
(74) Mandataire: Decobert, Jean-Pascal
(86) Numéro de dépôt international: PCT/FR2003/050093
(87) Numéro de publication internationale: WO 2004/045419

(56) Documents cités:
- EP-A- 0 834 279
- WO-A-02/19926
- WO-A-02/32320
- US-A- 4 580 573

## Description

La présente invention concerne un dispositif occlusif à destination médicale ou chirurgicale, ainsi qu'un dispositif d'occlusion vasculaire et une valve pour instrument chirurgical ou médical.

L'invention trouvera en particulier son application dans le domaine de la fabrication et de la mise en oeuvre de prothèses occlusives pour tous types de vaisseaux dans le corps humain ou animal, prothèses qui comprennent également des dispositifs transpariétaux et endovasculaires.

L'invention concerne également le domaine des instruments chirurgicaux ou médicaux et en particulier des instruments du type introducteurs utilisables lors d'interventions endovasculaires y compris percutanées et/ou transpariétales qui nécessitent la présence d'éléments d'obturation aptes à assurer l'étanchéité de l'introducteur.

La qualité de l'occlusion est un problème constant selon l'état de la technique actuel, que cela soit dans le domaine des prothèses vasculaires ou pour la constitution de valves.

On connaît du document WO-A-0219926, sur lequel est basée la forme en deux parties de la revendication 1, un dispositif d'occlusion vasculaire qui comporte deux organes expansibles pour sa fixation par appui sur deux portions de la paroi du vaisseau, une partie intermédiaire qui est déformable en torsion à un degré ajustable selon la position relative des deux organes expansibles. Une zone de striction maximale est ainsi créée définissant le degré d'occlusion.

Selon ce document, la réalisation d'une obturation totale ou partielle est formée par l'intermédiaire d'une déformation en torsion d'un élément.

Cette technique permet une grande facilité d'intervention et une possibilité de réglage très fin du degré d'obturation.

Il existe cependant un besoin d'améliorer encore l'étanchéité permise par ce type de dispositif.

La présente invention apporte une solution à ce problème en adjoignant d'autres éléments d'occlusion aptes à coopérer avec l'élément déformable en torsion.

De façon préférée mais non limitative, l'invention offre également l'avantage de proposer des moyens d'étanchéité additionnels sous forme de joints applicables sur la paroi d'un vaisseau éventuellement en combinaison avec un voile d'obturation.

D'autres buts et avantages apparaîtront au cours de la description qui suit d'un mode préféré de réalisation de l'invention qui n'est cependant pas limitatif.

La présente invention concerne un dispositif occlusif à destination médicale ou chirurgicale comportant un élément cylindrique creux déformable en torsion axiale pour créer une zone de striction, caractérisé par le fait qu'il comporte deux organes d'obturation solidaires de la paroi interne de l'élément cylindrique en préservant un passage et agencés pour s'appliquer l'un contre l'autre pour boucher le passage lorsque l'élément cylindrique est déformé en torsion.

Dans des modes de réalisation préférés, ce dispositif occlusif est tel que :
- les deux organes d'obturation sont solidaires de deux zones distinctes de la longueur de l'élément cylindrique.
- les organes d'obturation ont une section transversale en croissant de lune.
- les organes d'obturation sont solidaires de deux zones de la paroi de l'élément cylindrique diamétralement opposées.
- les organes d'obturation sont constitués en matériau polymère.
- il présente deux parties d'extrémité, encadrant l'élément cylindrique et dont la position angulaire relative détermine la torsion dudit élément cylindrique.
- l'élément cylindrique a une section circulaire.
- les organes d'obturation s'appliquent l'un contre l'autre par l'une de leurs faces latérales.

L'invention concerne également un dispositif d'occlusion vasculaire caractérisé par le fait qu'il comporte un dispositif occlusif selon l'invention.

A titre avantageux, ce dispositif d'occlusion vasculaire peut se présenter suivant les variantes énoncées ci-après :
- il présente deux parties d'extrémité, encadrant l'élément cylindrique et dont la position angulaire relative détermine la torsion dudit élément cylindrique, lesdites parties d'extrémité comportant des moyens de fixation sur la paroi d'un vaisseau.
- les moyens de fixation sont des organes expansibles.
- il comporte un joint sur la surface extérieure d'au moins un organe expansible, ledit joint étant apte à s'appliquer sur la paroi d'un vaisseau.
- il présente un voile périphérique d'obturation s'étendant entre une extrémité d'au moins un organe d'obturation et la bordure de l'organe expansible correspondant.
- il comporte un guide amovible orienté suivant l'axe de l'élément cylindrique et traversant le passage.
- il comporte une gaine amovible intercalée entre la paroi des organes d'obturation et la paroi externe du guide.
- il comporte un fourreau amovible entourant le dispositif occlusif.

L'invention concerne également une valve pour instrument chirurgical ou médical comportant un passage obturable et caractérisée par le fait qu'elle comporte un dispositif occlusif selon l'invention.

A titre préféré, la valve est telle que l'élément cylindrique est déformable en torsion par deux bagues chacune solidaires d'une extrémité de l'élément cylindrique.

Les dessins ci-joints sont donnés à titre d'exemples et ne sont pas limitatifs de l'invention. Ils représentent seulement un mode de réalisation de l'invention et permettront de la comprendre aisément.
La figure 1 est une vue générale du dispositif de l'invention pour une application à l'occlusion vasculaire.
La figure 2 illustre une étape de mise en place d'un dispositif d'occlusion vasculaire de façon transpariétale.
La figure 3 montre un exemple de résultat final d'occlusion transpariétale obtenu par le dispositif de l'invention.
La figure 4 est une vue en coupe selon les lignes C-C de la figure 5 du dispositif de l'invention.
La figure 5 est une vue de côté du dispositif intégré dans un fourreau.
La figure 6 en est une vue en coupe selon les lignes D-D.
La figure 7 montre une étape de mise en place du dispositif de l'invention.
La figure 8 en illustre une vue en coupe suivant les lignes E-E.
La figure 10 est une vue de côté du dispositif de l'invention dans un mode préféré de réalisation avec des voiles d'obturation.

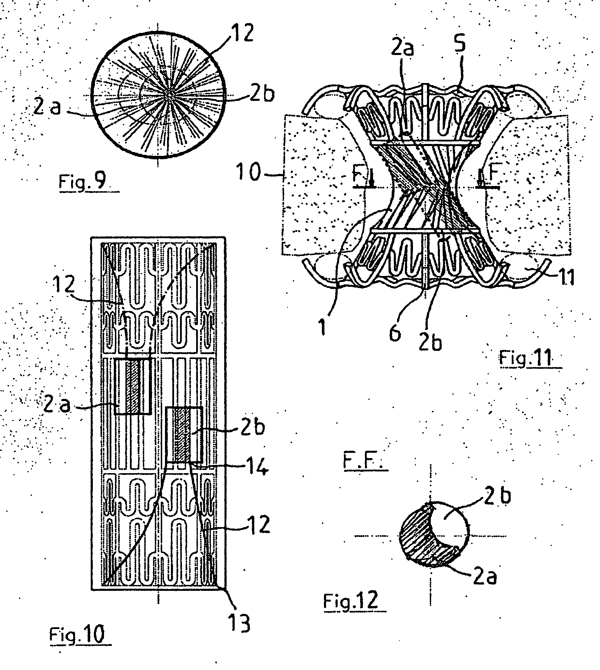

La figure 9 en est une vue de dessous.
La figure 11 illustre un dispositif d'occlusion vasculaire mis en place au travers de la paroi d'un vaisseau et la figure 12 en est une vue en coupe suivant les lignes F-F sans l'élément cylindrique.

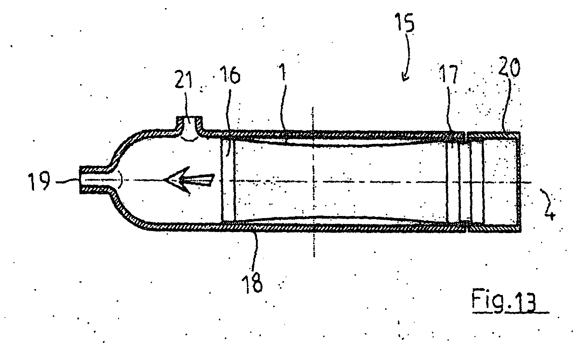

La figure 13 montre un mode de réalisation d'application du dispositif occlusif de l'invention à une valve pour instrument chirurgical ou médical de type introducteur.

Le dispositif occlusif selon l'invention peut être utilisé dans différents domaines d'application médicale ou chirurgicale.

On décrira plus particulièrement dans la suite de la description un mode de réalisation appliquant le dispositif occlusif à la formation d'un dispositif d'occlusion vasculaire ainsi qu'un mode de réalisation de l'invention appliquant le dispositif occlusif à des valves pour instruments chirurgicaux ou médicaux.

D'une façon générale, le dispositif selon l'invention comporte un élément cylindrique creux 1 déformable en torsion axiale d'axe 4, cette déformation permettant de créer une zone de striction avantageusement maximale vers le milieu de la longueur de l'élément cylindrique creux 1 bien que ce cas ne soit pas limitatif.

L'élément cylindrique peut être déformé en torsion en modifiant la position angulaire relative de ses extrémités.

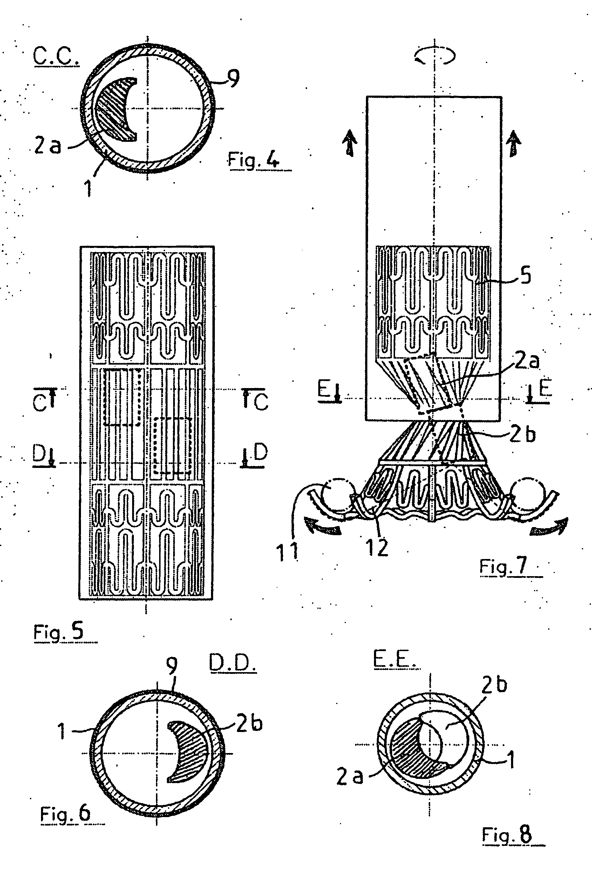

Le dispositif occlusif comporte en outre deux organes d'obturation 2a, 2b par exemple présentés aux figures 5 et 10 et solidaires de la paroi interne de l'élément cylindrique 1.

Les organes d'obturation 2a, 2b préservent un passage 3 au travers de l'élément cylindrique creux 1 en position de repos.

Par ailleurs, les organes d'obturation 2a, 2b sont avantageusement solidaires de deux zones distinctes sur la longueur de l'élément cylindrique 1.

Les figures 4 et 6 montrent un exemple de section transversale des organes d'obturation 2a, 2b, sous forme de croissants de lune aptes à définir une portion en arc de cercle constituant une paroi pour le passage résiduel 3.

Les organes d'obturation 2a, 2b peuvent être constitués en matériau polymère présentant des propriétés de mémoire de forme ou non.

Les organes d'obturation 2a, 2b sont disposés sur la paroi interne de l'élément cylindrique 1 de façon à ce que, lors de la déformation en torsion de l'élément cylindrique 1, ils s'appliquent l'un contre l'autre pour boucher le passage 3.

Un exemple de configuration en position obturée est présenté en figure 11.

La figure 12 montre clairement que, dans cette configuration relative, les organes d'obturation 2a, 2b se complètent pour obturer le passage 3.

Dans le cas représenté, l'application de deux organes d'obturation 2a, 2b s'effectue par l'un de leurs flancs ou encore faces latérales ici orientées transversalement relativement à l'axe 4.

L'application s'effectue donc en superposition suivant l'axe 4, par les surfaces en croissant de lune.

Eventuellement, une légère déformation en compression des organes d'obturation 2a, 2b lorsqu'ils s'appliquent est possible.

En outre, on peut agencer les organes 2a, 2b pour qu'ils s'appliquent l'un contre l'autre sur au moins une portion de leur surface longitudinale (orientée suivant l'axe 4).

On adapte la position relative des organes d'obturation 2a, 2b sur la paroi interne de élément cylindrique 1 en fonction de la déformation souhaitée pour l'élément cylindrique 1 jusqu'à parvenir à l'application des deux organes d'obturation 2a, 2b.

En particulier, il est possible de faire en sorte que les organes d'obturation 2a, 2b soient en position diamétralement opposée sur la paroi de l'élément cylindrique 1 en position de repos tel que cela ressort des figures 4 à 6.

Une rotation relative des deux extrémités de l'élément cylindrique d'une amplitude angulaire prédéterminée assure la mise en contact des flancs des deux organes d'obturation 2a, 2b.

On décrit ci-après plus précisément un mode de réalisation du dispositif occlusif pour une application à un dispositif d'occlusion vasculaire.

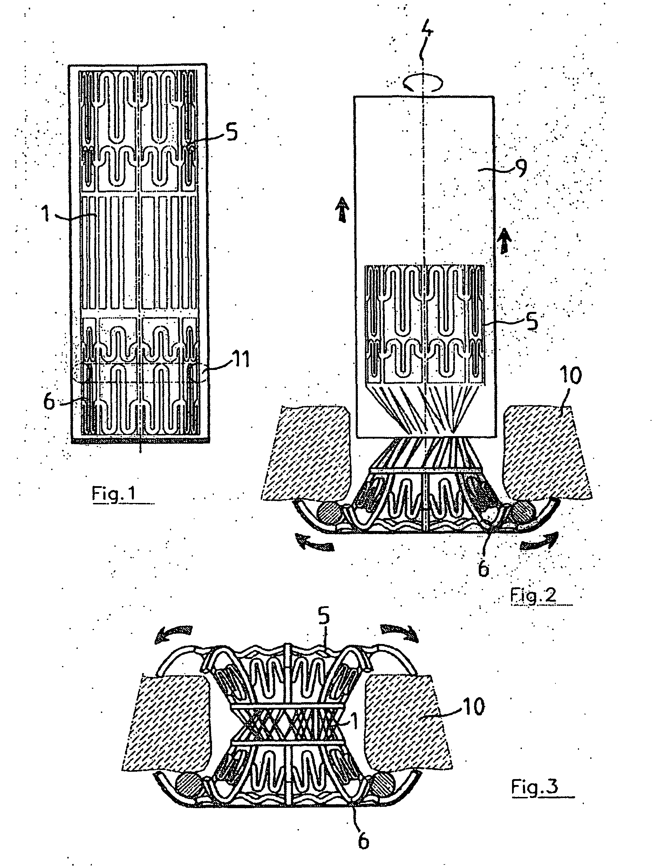

Dans ce cadre, il est fait référence aux figures 1 à 12 présentant un mode particulier de réalisation suivant cette application.

La figure 1 montre en détail un exemple de structure que peut présenter l'élément cylindrique 1. En particulier, l'élément 1 peut se présenter sous la forme d'une armature métallique, par exemple à base de Nitinol ® et présentant trois zones distinctes. La première zone, centrale, constitue l'élément cylindrique 1 en lui-même et est apte à se déformer en torsion tel que cela apparaît en figure 2 et 3. Autour de l'élément cylindrique 1, deux organes expansibles 5 sous forme d'armatures auto expansibles sont représentés et pourront se présenter suivant une configuration telle qu'actuellement utilisée dans le domaine des prothèses endovasculaires. Les organes expansibles 5, 6 ont des propriétés de mémoire de forme aptes à en assurer une déformation par déploiement lorsqu'ils sont libérés.

Cette libération s'effectue par l'intermédiaire d'un fourreau 9 entourant l'ensemble du dispositif avant la mise en place par le praticien. L'élément cylindrique 1 et les organes expansibles 5, 6 sont enveloppés dans le fourreau 9 en position de repos.

Au cours de l'implantation, le praticien retire progressivement le fourreau 9 de façon à libérer d'abord un premier organe expansible pour son application sur la paroi d'un vaisseau 10.

Ce retrait peut être opéré avec l'aide d'un élément pousseur sous forme d'un élément allongé cylindrique creux, apte, par son épaisseur, à s'appliquer sur le bord du dispositif d'occlusion pour exercer un effort contraire au retrait du fourreau 9 et immobiliser le dispositif d'occlusion durant ce retrait.

A cet instant, le dispositif d'occlusion vasculaire est partiellement positionné, mais l'organe expansible 5 reste dans le fourreau 9. La rotation du fourreau 9 par le praticien assure la mise en torsion de l'élément cylindrique 1 et la formation d'une zone de striction, tel que cela est représenté en figure 2.

Lorsque le degré de striction souhaité est obtenu (il est facilement réglable suivant l'amplitude de la rotation opérée par le praticien) l'autre organe expansible 5 est libéré du fourreau 9 par coulissement (toujours éventuellement en combinaison avec l'action d'un pousseur). Cette libération assure son déploiement et son application sur la paroi vasculaire 10.

Les figures 2 et 3 montrent plus particulièrement une application transpariétale au présent dispositif d'occlusion vasculaire. Dans ce cadre, c'est l'organe expansible 6 qui s'applique sur la paroi interne et l'organe expansible 5 sur la paroi externe.

Le fourreau 9 reçoit dans son volume intérieur l'ensemble formé par l'élément cylindrique 1 et les organes expansibles 5, 6.

En outre, l'élément cylindrique 1 reçoit lui-même, dans son volume intérieur, par fixation sur sa paroi interne, les organes d'obturation 2a, 2b, qui préservent cependant, un passage résiduel 3 suivant l'axe 4 du dispositif.

Par exemple, le passage 3 est apte à recevoir un guide utilisable lors des manipulations.

A titre préféré, l'étanchéité ainsi obtenue par l'intermédiaire du dispositif occlusif intégré dans le dispositif d'occlusion vasculaire est complétée par des moyens additionnels.

Plus particulièrement, un joint 11 s'appliquant sur la périphérie externe d'au moins un des organes expansibles 6, peut être constitué. On choisira un joint 11, par exemple de forme torique, et constituant une matière suffisamment déformable pour suivre les déformations de l'organe 6 lors de son déploiement.

Le joint 11 s'applique, par ce déploiement, sur la paroi du vaisseau 10.

Toujours à titre complémentaire à un dispositif occlusif, le dispositif d'occlusion vasculaire peut comprendre au moins un voile 12 tel qu'apparaissant en figure 10. En position de repos, le voile 12 a sensiblement une forme tronconique circulaire, éventuellement légèrement courbée, et s'étent entre une extrémité 14 de l'organe d'obturation et la bordure 13 de l'organe expansible 6 situé du même côté. Une telle situation est visible en figure 10 ainsi qu'en figure 9 en vue de dessous.

En constituant un tel voile 12 de façon continue, on forme un effet « entonnoir » évitant toute fuite de flux sanguin en dehors de la zone délimitée par le passage 3.

Lors du déploiement de l'organe expansible 6, le voile 12 en suit la déformation en corolle.

On décrit ci-après plus précisément un mode de réalisation du dispositif occlusif de l'invention pour une application à des valves pour instruments chirurgicaux et médicaux.

En particulier, la figure 13 illustre la formation d'une telle valve 15 intégrable ou rapportable sur un instrument d'introduction dans le corps.

A cet effet, la valve 15 comprend une partie d'enveloppe 18 apte à recevoir dans son volume intérieur un dispositif occlusif comprenant un élément cylindrique 1.

La valve 15 comprend en outre une extrémité proximale présentant une embouchure 19 pour le passage d'éléments au cours de l'introduction, ainsi qu'une ouverture additionnelle 21.

L'extrémité distale 20 de la valve 15 peut recevoir un élément de valve additionnel et/ou un simple organe de réglage angulaire.

Suivant cette application, l'élément cylindrique 1 est entouré de bagues 16, 17 dont la position angulaire relative est réglable de façon à assurer la mise en torsion de l'élément 1.

Bien que non représenté, l'élément 1 reçoit dans son volume intérieur les organes d'obturation 2a, 2b.

Suivant l'exemple, la rotation de la bague 17 engendrée par manipulation de l'extrémité distale 20 de la valve 15 modifie la position angulaire relative des bagues 16, 17 et assure une déformation en torsion de l'élément 1. Cette déformation en torsion engendre une modification de la position relative à des organes d'obturation 2a, 2b jusqu'à obtenir leur application apte à boucher le passage 3.

On peut ainsi ouvrir ou fermer totalement ou non le passage 3 en jouant sur la position de la bague 17, et ce alors que la bague 16 est fixée.

Bien entendu, ce mode de réalisation n'est qu'indicatif et des variantes peuvent être envisagées.

En particulier, la bague 16 peut être constituée mobile en rotation alors que la bague 17 pourrait être formée de façon fixe. En outre, un mouvement additionnel de rapprochement ou d'éloignement des bagues 16, 17 peut être opéré, par exemple par l'intermédiaire d'une liaison de type glissière hélicoïdale entre l'enveloppe 18 de la valve 15 et la bague 17.

Dans le cadre de cette application, l'élément cylindrique 1 comporte une paroi étanche et pourra être constitué notamment en une matière textile tissée ou non, ou encore en matériau polymère tel du P.T.F.E. (Poly Tétra Fluoro Ethylène).

### REFERENCES

1. Elément cylindrique
2a, 2b. Organes d'obturation
3. Passage
4. Axe
5. Organe expansible
6. Organe expansible
9. Fourreau
10. Paroi vasculaire
11. Joint
12. Voile
13. Bordure
14. Extrémité de l'organe d'obturation
15. Valve
16. Bague
17. Bague
18. Enveloppe
19. Embouchure
20. Extrémité distale
21. Ouverture

## Revendications

1. Dispositif occlusif à destination médicale ou chirurgicale comportant un élément cylindrique (1) creux déformable en torsion axiale pour créer une zone de striction, **caractérisé par le fait**
**qu'**il comporte deux organes d'obturation (2a, 2b) solidaires de la paroi interne de l'élément cylindrique (1) en préservant un passage (3) et agencés pour s'appliquer l'un contre l'autre pour boucher le passage (3) lorsque l'élément cylindrique (1) est déformé en torsion.

2. Dispositif selon la revendication 1, **caractérisé par le fait**
**que** les deux organes d'obturation (2a, 2b) sont solidaires de deux zones distinctes de la longueur de l'élément cylindrique (1).

3. Dispositif selon la revendication 1 ou 2 **caractérisé par le fait**
**que** les organes d'obturation (2a, 2b) ont une section transversale en croissant de lune.

4. Dispositif selon l'une quelconque des revendications 1 à 3, **caractérisé par le fait**
**que** les organes d'obturation (2a, 2b) sont solidaires de deux zones de la paroi de l'élément cylindrique (1) diamétralement opposées.

5. Dispositif selon l'une quelconque des revendications 1 à 4,
**caractérisé par le fait**
**que** les organes d'obturation (2a, 2b) sont constitués en matériau polymère.

6. Dispositif selon l'une quelconque des revendications 1 à 5, **caractérisé par le fait**
**qu'**il présente deux parties d'extrémité, encadrant l'élément cylindrique (1) et dont la position angulaire relative détermine la torsion dudit élément cylindrique (1).

7. Dispositif selon l'une quelconque des revendications 1 à 6, **caractérisé par le fait**
**que** l'élément cylindrique (1) a une section circulaire.

8. Dispositif selon l'une quelconque des revendications 1 à 7, **caractérisé par le fait**
**que** les organes d'obturation (2a, 2b) s'appliquent l'un contre l'autre par l'une de leurs faces latérales.

9. Dispositif d'occlusion vasculaire, **caractérisé par le fait**
**qu'**il comporte un dispositif occlusif selon l'une quelconque des revendications 1 à 8.

10. Dispositif selon la revendication 9, **caractérisé par le fait**
**qu'**il présente deux parties d'extrémité, encadrant l'élément cylindrique (1) et dont la position angulaire relative détermine la torsion dudit élément cylindrique (1), lesdites parties d'extrémité comportant des moyens de fixation sur la paroi d'un vaisseau.

11. Dispositif selon la revendication 10, **caractérisé par le fait**
**que** les moyens de fixation sont des organes expansibles (5, 6).

12. Dispositif selon la revendication 11, **caractérisé par le fait**
**qu'**il comporte un joint (11) sur la surface extérieure d'au moins un organe expansible (5, 6), ledit joint (11) étant apte à s'appliquer sur la paroi d'un vaisseau.

13. Dispositif selon l'une quelconque des revendications 11 ou 12, **caractérisé par le fait**
**qu'**il présente un voile (12) périphérique d'obturation s'étendant entre une extrémité d'au moins un organe d'obturation et la bordure (13) de l'organe expansible (5, 6) correspondant.

14. Dispositif selon l'une quelconque des revendications 9 à 13, **caractérisé par le fait**
**qu'**il comporte un guide (7) amovible orienté suivant l'axe (4) de l'élément cylindrique (1) et traversant le passage (3).

15. Dispositif selon la revendication 14, **caractérisé par le fait**
**qu'**il comporte une gaine (8) amovible intercalée entre la paroi des organes d'obturation (2a, 2b) et la paroi externe du guide (7).

16. Dispositif selon l'une quelconque des revendications 9 à 15, **caractérisé par le fait**
**qu'**il comporte un fourreau (9) amovible entourant le dispositif occlusif.

17. Valve (15) pour instrument chirurgical ou médical, comportant un passage obturable, **caractérisée par le fait**
**qu'**elle comporte un dispositif occlusif selon l'une quelconque des revendications 1 à 8.

18. Valve (15) selon la revendication 17, **caractérisée par le fait**
**que** l'élément cylindrique (1) est déformable en torsion par deux bagues (16, 17) chacune solidaire d'une extrémité de l'élément cylindrique (1).

## Claims

1. Occlusive device for medical or surgical use, comprising a hollow cylindrical element (1) that can be twisted according to its axis to create a striction zone, **characterised in that**
it comprises two obturation elements (2a, 2b) integral to the inner wall of the cylindrical element (1), leaving a passage (3) and arranged to press against each other to block the passage (3) when the cylindrical element (1) is twisted.

2. Device according to claim 1, **characterised in that**
the two obturation elements (2a, 2b) are integral to two distinct areas of the length of the cylindrical element (1).

3. Device according to claim 1 or 2, **characterised in that**
the obturation elements (2a, 2b) have a crescent-shaped cross section.

4. Device according to any of claims 1 to 3, **characterised in that**
the two obturation elements (2a, 2b) are integral to two diametrically opposed areas of the wall of the cylindrical element (1).

5. Device according to any of claims 1 to 4, **characterised in that**
the obturation elements (2a, 2b) are made from a polymeric material.

6. Device according to any of claims 1 to 5, **characterised in that**
there are two end parts, surrounding the cylindrical element (1) and whose angular position determines the torsion of said cylindrical element (1).

7. Device according to any of claims 1 to 6, **characterised in that**
the cylindrical element (1) has a circular cross section.

8. Device according to any of claims 1 to 7, **characterised in that**
the obturation elements (2a, 2b) are applied one against each other by means of one of their lateral surfaces.

9. Vascular occlusion device, **characterised in that**
it comprises an occlusive device according to any of claims 1 to 8.

10. Device according to claim 9, **characterised in that**
it possesses two end parts, surrounding the cylindrical element (1) and whose relative angular position determines the torsion of said cylindrical element (1), said end parts possessing means of attachment to the wall of a vessel.

11. Device according to claim 10, **characterised in that**
the attachment systems are expanding elements (5, 6).

12. Device according to claim 11, **characterised in that**
it possesses a seal (11) on the outer surface of at least one of the expanding elements (5, 6), said seal (11) being appropriate for application to the wall of a vessel.

13. Device according to either of claims 11 or 12, **characterised in that**
it presents a peripheral obturation web (12) extending from one end of at least one obturation element and the edge (13) of the corresponding expanding element (5, 6).

14. Device according to any of claims 9 to 13, **characterised in that**
it comprises a removable guide (7) positioned according to the axis (4) of the cylindrical element (1) and crossing the passage (3).

15. Device according to claim 14, **characterised in that**
it possesses a removable sheath (8) inserted between the wall of the obturation elements (2a, 2b) and the external wall of the guide (7).

16. Device according to any of claims 9 to 15, **characterised in that**
it comprises a removable sleeve (9) surrounding the occlusive device.

17. Valve (15) for surgical or medical instrument, comprising a closeable passage, **characterised in that**
it comprises an occlusive device according to any of claims 1 to 8.

18. Valve (15) according to claim 17, **characterised in that**
the cylindrical element (1) can be twisted by means of two rings (16, 17), each of which is integral to one end of the cylindrical element (1).

## Patentansprüche

1. Verschlussvorrichtung für medizinische oder chirurgische Zwecke bestehend aus einem hohlen, zylindrischen Element (1), das durch Torsion um die Längsachse eine Querschnittsverengung bilden kann, **gekennzeichnet dadurch, dass** es zwei mit der Innenwand des zylindrischen Elements (1) verbundene Verschlusselemente (2a, 2b) besitzt, die so gestaltet sind, dass ein Durchgang (3) gebildet wird, sie sich jedoch, wenn das zylindrische Element (1) durch Torsion verformt wird, aneinander anlegen können, um den Durchgang (3) zu verschließen.

2. Vorrichtung gemäß Anspruch 1, **gekennzeichnet dadurch, dass** die beiden Verschlusselemente (2a, 2b) mit zwei Zonen verbunden sind, deren Lage in Längsrichtung des zylindrischen Elements (1) unterschiedlich ist.

3. Vorrichtung gemäß Anspruch 1 oder 2, **gekennzeichnet dadurch, dass** die Verschlusselemente (2a, 2b) einen halbmondförmigen Querschnitt besitzen.

4. Vorrichtung gemäß einem der Ansprüche 1 bis 3, **gekennzeichnet dadurch, dass** die Verschlusselemente (2a, 2b) mit zwei sich diametral gegenüberliegenden Zonen auf der Wand des zylindrischen Elements (1) verbunden sind.

5. Vorrichtung gemäß einem der Ansprüche 1 bis 4, **gekennzeichnet dadurch, dass** die Verschlusselemente (2a, 2b) aus Polymerwerkstoff besteht.

6. Vorrichtung gemäß einem der Ansprüche 1 bis 5, **gekennzeichnet dadurch, dass** sie zwei Endstücke besitzt, die das zylindrische Element (1) einschließen und deren relative Winkelstellung die Torsion des genannten zylindrischen Elements (1) bestimmt.

7. Vorrichtung gemäß einem der Ansprüche 1 bis 6, **gekennzeichnet dadurch, dass** das zylindrische Element (1) einen kreisförmigen Querschnitt besitzt.

8. Vorrichtung gemäß einem der Ansprüche 1 bis 7, **gekennzeichnet dadurch, dass** sich die Verschlusselemente (2a, 2b) mit jeweils einer ihrer Seitenflächen aneinander anlegen.

9. Verschlussvorrichtung für Blutgefäße, **gekennzeichnet dadurch, dass** sie eine Verschlussvorrichtung gemäß einem der Ansprüche 1 bis 8 besitzt.

10. Vorrichtung gemäß Anspruch 9, **gekennzeichnet dadurch, dass** sie zwei Endstücke besitzt, die das zylindrische Element (1) einschließen und deren relative Winkelstellung die Torsion des genannten zylindrischen Elements (1) bestimmt, wobei die Endstücke Mittel zur Fixierung an der Wandung eines Blutgefäßes besitzen.

11. Vorrichtung gemäß Anspruch 10, **gekennzeichnet dadurch, dass** die Fixiermittel expandierbare Organe (5, 6) sind.

12. Vorrichtung gemäß Anspruch 11, **gekennzeichnet dadurch, dass** sie eine Dichtung (11) an der Außenseite mindestens eines der expandierbaren Organe (5, 6) besitzt und diese Dichtung (11) sich an die Wandung eines Blutgefäßes anlegen kann.

13. Vorrichtung gemäß einem der Ansprüche 11 oder 12, **gekennzeichnet dadurch, dass** sie an ihrem Umfang eine verschließende Einlage (12) besitzt, die sich von mindestens einem Ende eines Verschlusselements zum Rand (13) des entsprechenden expandierbaren Organs (5, 6) erstreckt.

14. Vorrichtung gemäß einem der Ansprüche 9 bis 13, **gekennzeichnet dadurch, dass** sie eine abnehmbare, in Längsrichtung (4) des zylindrischen Elements (1) gerichtete und durch den Durchgang (3) hindurchtretende Führung (7) besitzt.

15. Vorrichtung gemäß Patentanspruch 14, **gekennzeichnet dadurch, dass** sie ein abnehmbares Schutzrohr (8) zwischen der Wand der Verschlusselemente (2a, 2b) und der Außenwand der Führung (7) besitzt.

16. Vorrichtung gemäß einem der Ansprüche 9 bis 15, **gekennzeichnet dadurch, dass** sie eine die Verschlussvorrichtung umgebende, abnehmbare Hülse (9) besitzt.

17. Klappe (15) für chirurgische oder medizinische Instrumente mit einem verschließbaren Durchgang, **gekennzeichnet dadurch, dass** er eine Verschlussvorrichtung gemäß einem der Ansprüche 1 bis 8 besitzt.

18. Klappe (15) gemäß Anspruch 17, **gekennzeichnet dadurch, dass** das zylindrische Element (1) mit Hilfe zweier Ringe (16, 17), der jeder mit einem Ende des zylindrischen Elements (1) verbunden ist, durch Torsion verformbar ist.
